# EUROPEAN PATENT SPECIFICATION

(11) **EP 2 229 114 B1**
(45) Date of publication and mention of the grant of the patent: **08.06.2011**
(21) Application number: 09700085.5
(22) Date of filing: 05.01.2009
(51) Int. Cl.: A61B 18/14, A61B 18/16

(54) **HIGH FREQUENCY GENERATOR FOR ELECTROSURGICAL CUTTING**
HOCHFREQUENZGENERATOR FÜR ELEKTROCHIRURGISCHE SCHNEIDEVORGÄNGE
GÉNÉRATEUR HAUTE FRÉQUENCE POUR INCISION ÉLECTROCHIRURGICALE

(30) Priority: 03.01.2008 DE 102008003475
(43) Date of publication of application: 22.09.2010
(73) Proprietor: Celon AG medical instruments, 14513 Teltow (DE)
(72) Inventor: ROGGAN, André, 12163 Berlin (DE); STRAUSS, Timo, 10711 Berlin (DE)
(74) Representative: Eisenführ, Speiser & Partner
(86) International application number: PCT/EP2009/050043
(87) International publication number: WO 2009/083617

(56) References cited:
- EP-A- 1 849 425
- DE-A1- 3 608 833
- US-A- 6 039 732

## Description

The invention concerns a high frequency generator for the connection of an electrosurgical instrument for cutting body tissue by means of an arc.

High frequency generators of that kind usually have an electrical output terminal to which an electrosurgical instrument can be connected and an input terminal by way of which it can be connected to a current or voltage source. In that case power control serves for controlling the electrical power delivered by way of the output terminal.

Devices of that kind are basically already known from for example DE 195 00 219, DE 41 35 185, DE 41 26 609, DE 36 22 337, DE 34 20 340, DE 29 46 728, DE 35 30 335 or DE 32 28 136. A device according to the preamble of claim 1 is known from US 6039732.

In electrosurgical cutting, in particular in the case of endoscopic interventions or in polypectomy, there is the danger that the respective electrosurgical cutting electrode being used penetrates into the tissue to an excessive depth because the operator cannot sufficiently sensitively guide the electrosurgical instrument.

To reduce that risk and to permit electrosurgical cutting guidance which is as controlled as possible known high frequency generators have a cyclically implemented power delivery when providing the electrical power required for electrosurgical cutting. That cyclic power delivery involves cutting intervals which are comparatively short in terms of time and which involve a relatively high level of electrical power, and comparatively long coagulation or pause intervals in which no or a low level of electrical power is delivered.

High frequency generators designed for electrosurgical cutting instruments generally have initial cutting assistance or support, that is to say to produce an arc firstly a very high level of power is delivered in order to rapidly dry out the body tissue bearing against the cutting electrode, and thereby rapidly to achieve a high tissue impedance necessary to produce an arc and thus the voltage required for firing the arc. Thereafter the power can be reduced, with the arc being maintained. In actual fact the arc can never be permanently maintained with an applied ac voltage, but at the latest collapses at each zero crossing of the ac voltage. What is meant however in regard to the term which is also used hereinafter, 'maintaining the arc', is that the tissue drying which is achieved means that the impedance (and therewith also the voltage) remain so high that, in each period of the ac voltage, the peak voltage reaches the value required for igniting an arc.

At the beginning of application of the high frequency current however, the excessively low impedance means that ignition of an arc does not happen straightaway so that initial cutting support is appropriate. Such initial cutting support is appropriate in particular when using cutting electrodes of large area such as for example polypectomy snare loops in order to permit the large area to dry out quickly and thus to ensure a rapid initial cutting characteristic.

Particularly in the case of endoscopic mucosal resection (EMR) or polypectomy, because of the large-area snare loop cutting electrodes, it is often not possible to achieve the high impedance required for ignition of an arc with only one pulse with a high electrical power level for initial cutting support because the thermal energy introduced into the tissue is not sufficient to increase the impedance and therewith also the voltage in the region of the cutting electrode to such an extent that an arc can be ignited.

For that reason in known high frequency generators the power delivery is implemented cyclically in accordance with a fixed pulse sequence:

Firstly an initial cutting pulse is delivered, involving a very high power, and then, when the arc ignites, a pulse of reduced power is produced to maintain the arc, and finally a pause interval.

The length of the pause interval is in that case so selected that the surgical instrument, as already described, can be guided in more controlled fashion. It is precisely when for example with large-area electrodes a large number of initial cut-supporting pulses is required in order to ignite an arc that the waiting time required for that purpose can be perceived by the operator as being a nuisance. The object of the invention is to provide a high frequency generator of the kind set forth in the opening part of this specification, which provides a better initial cut characteristic.

In accordance with the invention that object is attained by a high frequency generator of the kind set forth in the opening part of this specification which has an arc detector and a power control which is so adapted that initially for a phase for initial cutting support it causes the delivery of a high output power for initial cutting support and subsequently thereto
- either if ignition of an arc has occurred during the phase for initial cutting support, for a predetermined period of time of a cutting phase it causes the delivery of a power which is reduced in relation to the high power and subsequently for a predetermined period of time of a long pause interval it causes the delivery of no power or a low power at which no arc occurs,
- or if no ignition of an arc has occurred until the attainment of a predetermined maximum duration of the phase for initial cutting support it causes the delivery of no power or a low power for a predetermined period of time of a short pause interval.

The invention is based on the realisation that, for the situation where no arc has been ignited, a long pause or coagulation interval is not required and can therefore lead to unnecessary waiting times during the course of the treatment.

An important advantage enjoyed by the high frequency generator according to the invention over the state of the art is that the time which elapses until first ignition of the arc is shortened in comparison with high frequency generators in the state of the art.

More specifically high frequency generators in the state of the art provide a constant sequence of initially a phase for initial cutting support, then a cutting phase, and lastly a pause or coagulation interval, in which respect the presence of an arc has no influence on the length of the pause or coagulation interval.

In the case of the high frequency generator according to the invention the power delivered during the initial cutting support phase and the power delivered during the cutting phase and the power delivered during the coagulation or pause interval preferably respectively corresponds approximately to those powers which are already known from the state of the art. Suitable frequencies for the individual phases are also to be found in the state of the art.

Preferably the maximum power in the initial cutting support phase is about 500 watts and during the cutting phase it is preferably about 250 watts. The preferred frequency in that respect is preferably between 300 kHz and 2 MHz.

Preferably the predetermined maximum duration of the initial cutting support phase is about 50 ms. Preferably the predetermined period of time for the cutting phase is about 15 ms.

A typical period of time for the long coagulation or pause interval following a cutting phase is about 500 - 1000 ms while a suitable period of time for the short coagulation or pause interval directly following the initial cutting support phase (without cutting phase as no arc was ignited) is about 100 - 400 ms.

Besides the high frequency generator just disclosed the invention also concerns a method of electrosurgical cutting of body tissue, wherein the method firstly provides
the application of a high frequency current with a high power during an initial cutting support phase (wherein application of the high power takes place at most for the period of time of a predetermined maximum duration) and subsequently thereto either
- application of a high frequency current at a power which is reduced in comparison with the high power for a predetermined period of time of a cutting phase and subsequently thereto the application of a low or no power for the period of time of a predetermined long pause or coagulation interval if ignition of an arc has occurred during the application of the high frequency current at high power, or
- application of a high frequency current of a low or no power for a predetermined period of time of a short pause or coagulation interval if no ignition of an arc has occurred during application of the high frequency current at high power.

As the method according to the invention corresponds to an electrosurgical treatment of patients using the high frequency generator according to the invention the surgical use of the different variants of the high frequency generator according to the invention represents different variants of the method.

An embodiment of the invention will now be described in greater detail with reference to Figures 1 to 3 in which:
Figure 1 shows a high frequency generator with a connected electrical instrument,
Figure 2 shows a circuit diagram illustrating the principle of the high frequency generator,
Figure 3 shows an example of a pulse diagram of the pulse sequences outputted at the output of the high frequency generator.

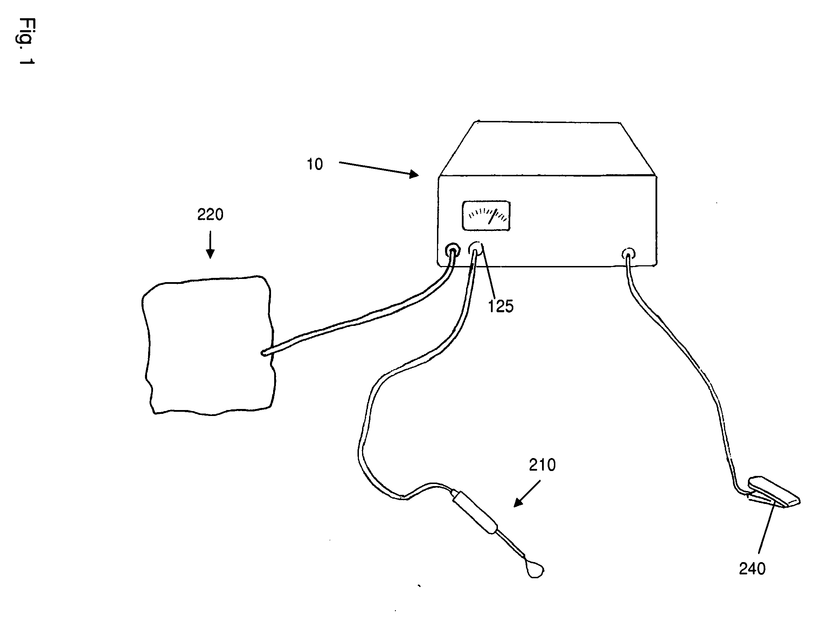

Figure 1 shows a high frequency generator 10 provided at the input side with a power cable for connection to a public mains system. The high frequency generator 10 is connected to a foot switch 240 serving to switch the high frequency generator 10 on and off. On the output side two output poles 125 of the high frequency generator 10 are connected by way of current lines to an electrode 210 of an electrosurgical instrument and a neutral electrode 220. The electrosurgical instrument in this case has a cutting electrode 210 which in the embodiment by way of example as shown in Figure 1 is in the form of a snare loop cutting instrument.

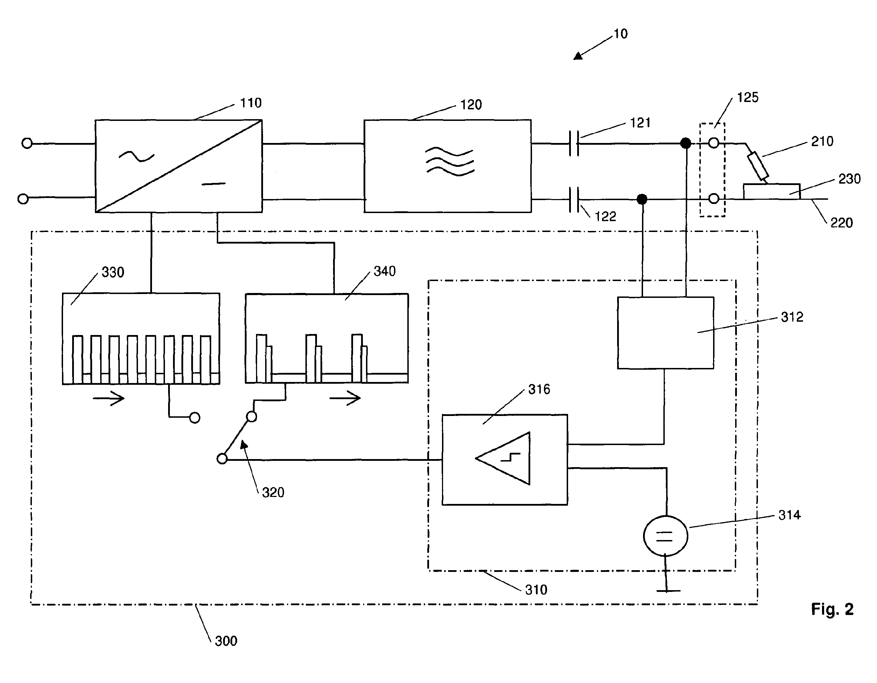

Figure 2 shows a circuit diagram illustrating the principle of a preferred embodiment of the high frequency generator according to the invention with an initial cutting performance which is improved over the state of the art. The illustrated embodiment is in particular also suitable for surgical interventions in the field of endoscopic mucosal resection (EMR) and polypectomy.

The high frequency generator 10 includes a clock-controllable power supply unit 110 which can be connected at the input side to the public ac supply mains and which at the output side is connected to the input of a high frequency generator module 120. The power supply unit 110 converts an ac voltage into a dc voltage which can be clock-controlled by means of at least one clock in such a way that pulse sequences involving pulses of different pulse lengths and of different pulse amplitudes are available at the output of the power supply unit 110. In other words the power delivery of the power supply unit 110 is clock-controlled and a succession of different power levels is outputted for the respective length of a corresponding time interval. The high frequency generator module 120 converts the dc voltage into an ac voltage at a frequency of between 0.3 and 2 MHz. The output of the high frequency generator module 120 is connected by way of two antifaradisation capacitors 121, 122 and by way of a two-pole electrical output terminal 125 of the high frequency generator 10 to an electrode 210 of an electrosurgical instrument and a neutral electrode 220. The two antifaradisation capacitors are intended to prevent the transmission of dangerous direct currents which occur upon ignition of an arc between the electrode 210 and tissue 230, to the patient.

The electrosurgical instrument generally comprises a cutting electrode 210 and a handle. In order to be able to electrosurgically cut tissue in the proximity of the cutting electrode 210 an arc must be produced between the electrode 210 and the body tissue. That is effected by a direct current of high electrical power, which is clock-controlled by means of at least one clock, being outputted at the output of the power supply unit 110 to the input of the high frequency generator module 120. Thereupon the high frequency generator module 120 outputs for the same period of time a high frequency current, that is to say high frequency alternating current, of high electrical power. The cutting electrode 210 of the electrosurgical instrument and the neutral electrode 220 are connected to the output of the high frequency generator module 120 by way of the two-pole electrical output terminal 125. Heating of the tissue 230 bearing against the cutting electrode 210 and thus rapid drying-out thereof are effected by the high frequency current outputted to the electrodes 210 and 220. As a result the impedance of the body tissue rises in the proximity of the electrode 210 and the voltage required for ignition of the arc is achieved. It is only with that arc that the operator is in a position to carry out a desired cut in the tissue 230 against the cutting electrode 210.

To maintain the arc required for electrosurgical cutting after a previously successfully ignited arc, a comparatively lower level of electrical power is sufficient.

Switching over from the high ignition power level to a lower power level for maintaining the arc is effected for example by a power control 300 which is disposed in the high frequency generator 10 and which includes an arc detector 310, a change-over switch 320, a first clock 330 and a second clock 340.

The arc detector 310 in turn includes an arc sensor 312, a reference signal source 314 and a comparator 316. The inputs of the arc sensor 312, as inputs of the arc detector 310, are connected at the output side downstream of the antifaradisation capacitors 121 and 122 to the poles of the high frequency generator module 120 and detect the presence of an arc. In that case the output of the arc sensor 312 is connected to the input of the comparator 316. The output of the reference signal source 314 is also connected to the input of the comparator 316. The output of the comparator 316, as the output of the arc detector 310, is connected to the input of the change-over switch 320, the two outputs of which are connected to the two clocks 330 and 340. The change-over switch 320 activates the clock 330 or 340 in dependence on the output signal of the arc detector 310.

The outputs of the clocks 330 and 340 are at the same time the outputs of the power control 300. The clock 330 causes the power supply unit to output direct current at a high power level (for initial cutting support) and to output direct current at a low power level for short pause intervals therebetween. No power may also be outputted in the pause interval.

The clock 340 causes the power supply unit to output direct current at a high power level (for initial cutting support) and - immediately thereafter, after ignition of the arc - to output direct current at a lower power level for maintaining the arc and finally to output direct current at a low power level for long pause intervals. In this case also alternatively no power may be outputted in the pause interval.

To detect the arc the arc sensor 310 detects for example a dc voltage applied across the electrodes 210 and 220. As a dc voltage measured there is a function of the rectified current at the cutting location, which is caused when cutting with a high frequency current, the arc detector 310 can thereby detect the presence of an arc. Arc detection on the basis of a dc voltage which is produced is known from DE 28 01 833.

The arc detector 310 can however also be adapted to analyse the spectral power distribution of the output of the generator 10 and in that case to compare two different frequency ranges to each other. The production of an arc can also be detected by an analysis of the 'higher harmonics'. The use of 'higher harmonics' is described in this context in greater detail in DE 41 26 607 A1.

As an alternative thereto the arc detector 310 can also be adapted to detect the presence of an arc by means of a photooptical element as the arc sensor 312. Such a detector is proposed in DE 25 04 280.

Arc detection can also be effected by the detection of a sudden change in impedance. In general terms the arc detector 310 can be designed in any desired fashion to detect the presence of an arc.

At any event the sensor 312 outputs a signal proportional to the measurement value, to the input of the comparator 316. The comparator 316 compares that output signal to the reference signal which is also at the input of the comparator 316 and which is outputted by the reference signal source 314. Comparison of the two values establishes whether an arc has been ignited at the electrosurgical instrument.

It will be appreciated that the individual components listed here of the arc detector 310 can also be embodied in a programmable microcontroller.

When an arc is present the arc detector 310 outputs a representative signal.

When no arc has occurred at the electrode 210 of the electrosurgical instrument then the arc detector 310 switches the change-over switch 320 to the first clock 330 for initial cutting support in order to prevent an unnecessarily long pause or coagulation interval and to reduce the time until ignition of an arc occurs.

When however an arc has occurred at the electrosurgical instrument then the arc detector 310 switches the change-over switch 320 over to the second clock 340 to implement a long pause or coagulation interval which is now necessary. In that case the clocks 330 and 340 of the power control 340 provide for clock control of the dc voltage output of the power supply unit 110 in such a way that the above-mentioned pulse sequences are outputted at the output of the power supply unit 110.

The power control 300 can also be entirely or partially implemented in a microcontroller in which the components 310, 320, 330 and 340 are programmed.

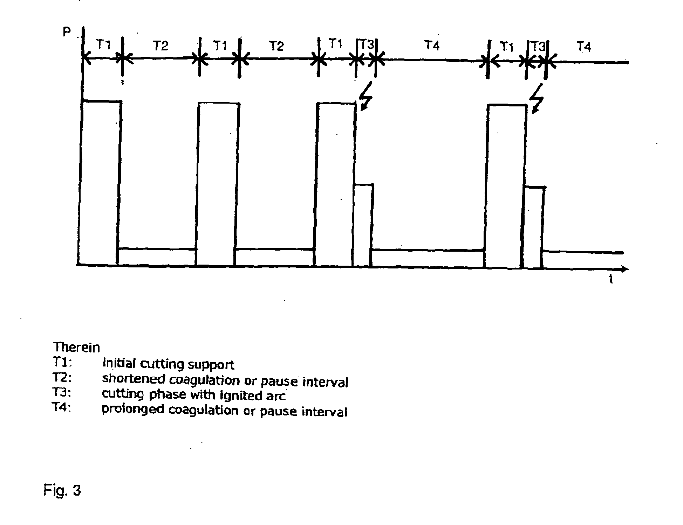

The power delivered by the high frequency generator 10 to the electrode 210 of the electrosurgical instrument is shown in detail in Figure 3 in a pulse diagram.

The time sequence shown in Figure 3 begins as soon as the physician actuates the foot switch 240 by depressing it. The foot switch 240 remains depressed until the end of the application.

The time intervals T1 which are shown in the pulse diagram and which involve a high power level P1 serve for initial cutting support. If the impedance of the tissue 230 is still not sufficient, that is to say the tissue 230 is not yet sufficiently dried out, the time interval T1 is followed by a time interval T2 as a shortened coagulation or pulse interval involving a low power level P3. The time intervals T1 and T2 can be repeated a plurality of times until an arc is ignited.

If ignition of an arc is detected during or after a time interval T1 a time interval T3 immediately follows. The time interval T3 is a cutting interval and serves to maintain an arc which was previously successfully ignited in the time interval T1, and has a lower power level than the time interval T1 because a lower power level is sufficient to maintain the arc. The time interval T3 is followed by a time interval T4 as a prolonged coagulation or pause interval involving a markedly lower power level P3. The time intervals T1, T3 and T4 are repeated as long as the cutting operation in the tissue 230 lasts, that is to say as long as the physician actuates the foot switch 240.

According to a further pulse diagram for a high frequency generator 10 according to the invention, at the beginning of the application, as shown in Figure 3, a high power P1 is delivered in an initial cutting support phase. The maximum duration T1 is predetermined for the initial cutting support phase. As no arc is ignited in the procedure illustrated by way of example during the first two initial cutting support phases, as in Figure 3, the high power P1 occurs until the end of the maximum duration T1. At the beginning of the application the arc detector 310 does not deliver a signal or at least not a signal representative of the existence of an arc. In addition the change-over switch 320 actuates the clock 330 at the beginning of the application.

The initial cutting support phase is followed by the short pause interval after the expiry of the maximum duration T1, as in Figure 3, for a predetermined short pause time T2. In the short pause interval the HF generator delivers a lower level of power P3. The lower power P3 can even be so low that it is substantially equal to zero. After the end of the short pause interval an initial cutting support phase is again effected, as already described with reference to Figure 3. Initial cutting support phases and short pause intervals alternate as long as no arc is ignited and the arc detector 310 thus does not output a signal representative of the presence of an arc.

Eventually, an arc ignites during the third initial cutting support phase. The arc detector 310 outputs the arc signal representative of the presence of the arc.

When the arc signal occurs the initial cutting support phase ends immediately and there then follows a cutting phase at reduced power P2, which is suitable for maintaining the arc. The reduced power P2 is higher than the low power P3 but lower than the high power P1. The initial cutting support phase ends as soon as the arc signal occurs after a time T-1 which can be shorter than the maximum duration T1. The cutting phase is of a predetermined time duration T3.

After the end of the cutting phase there now follows the long pause interval involving a long pause time T4 because, with the occurrence of the arc signal, the change-over switch 320 switched over from the clock 330 to the clock 340. With the end of the cutting phase the arc is extinguished and there is thus no arc signal. Nonetheless the clock 340 remains activated by the change-over switch 320. At the end of the long pause time T4 there again follows an initial cutting support phase, during which an arc is again ignited. The moment in the initial cutting support phase at which the arc ignites can vary. Therefore the duration T-1 of the initial cutting support phase can also vary.

## Claims

1. A high frequency generator (10) for the connection of an electrosurgical instrument (210) comprising
- an electrical output terminal (125) for an electrosurgical instrument,
- a current or voltage source (110) connected at least indirectly to the output terminal,
- an arc detector (310) which in operation delivers an arc signal (410) representative of an arc if an arc has ignited at the electrosurgical instrument, and
- a power control (300) for controlling the electrical power delivered by way of the output terminal (125), whereby the power control is adapted
- firstly to cause the delivery of a high output power (P1) during an initial cutting support phase (420) **characterised in that** the power control (300) is further adapted subsequently thereto
- either to cause the delivery of a power (P2) which is reduced in relation to the high power (P₁) to maintain the arc for a predetermined period of time (T3) of a cutting phase (440) if the arc signal (410) was outputted during the initial cutting support phase (420), and subsequently for a predetermined period of time (T4) of a long pause interval (450) to cause the delivery of no power or a lower power (P3) at which no arc occurs,
- or (430) to cause the delivery of no power or a low power at which no arc occurs, if no arc signal (410) was outputted until the attainment of a predetermined maximum duration (T1) of the initial cutting support phase (420) for a predetermined period of time (T2) of a pause interval shorter than said long pause interval.

2. A high frequency generator (10) according to claim 1 **characterised in that** the power control (300) is so adapted that the time duration of the long pause interval is between 500 and 1000 ms and the time duration of the short pause interval is between 100 and 400 ms.

3. A high frequency generator (10) according to claim 1 or claim 2 **characterised in that** the power control (300) is so adapted that the time duration of a cutting phase is 10 - 20 ms, preferably 15 ms.

4. A high frequency generator (10) according to one of claims 1 to 3 **characterised in that** the power control (300) is so adapted that the phase for initial cutting support ends in any case and goes into the cutting phase when the arc detector (310) detects the ignition of an arc.

5. A high frequency generator (10) according to claim 4 **characterised in that** the arc detector (310) is so adapted that it detects the ignition of an arc by the detection of a dc voltage.

6. A high frequency generator (10) according to one of claims 1 to 5 **characterised in that** the arc detector (310) detects the ignition of an arc from a characteristic configuration of the higher harmonics.

7. A high frequency generator (10) according to one of claims 1 to 6 **characterised in that** the power control (300) is so adapted that the maximum duration of the phase for initial cutting support is less than 50 ms.

8. A high frequency generator (10) according to one of claims 1 to 7 **characterised in that** the maximum duration of the phase for initial cutting support and/or the time duration of the cutting phase and/or the time duration of the long pause interval and/or the time duration of the short pause interval is/are adjustable.

9. A high frequency generator (10) according to one of claims 1 to 8 **characterised in that** the delivered power of the phase for initial cutting support and/or the delivered power of the cutting phase and/or the delivered power of the long pause interval and/or the delivered power of the short pause interval is/are adjustable.

## Patentansprüche

1. Hochfrequenzgenerator (10) zum Anschließen eines elektrochirurgischen Instrumentes (210) mit
- einem elektrischen Ausgangsanschluss (125) für ein elektrochirurgisches Instrument,
- einer Strom- oder Spannungsquelle (110), die wenigstens mittelbar mit dem Ausgangsanschluss verbunden ist,
- einem Lichtbogendetektor (310), der im Betrieb ein für einen Lichtbogen repräsentatives Lichtbogensignal (410) abgibt, wenn ein Lichtbogen am elektrochirurgischen Instrument gezündet hat, und
- einer Leistungssteuerung (300) zum Steuern der über den Ausgangsanschluss (125) abgegebenen elektrischen Leistung, wobei die Leistungssteuerung dazu ausgebildet ist,
- zunächst während einer Anschnittunterstützungsphase (420) die Abgabe einer hohen Leistung (P1) zu bewirken, **dadurch gekennzeichnet, dass** die Leistungssteuerung (300) weiter dazu ausgebildet ist, im Anschluss daran
- entweder die Abgabe einer gegenüber der hohen Leistung (P1) verminderen Leistung (P2) zu bewirken, um den Lichtbogen für eine vorbestimmte Zeitdauer (T3) einer Schnittphase (440) aufrecht zu erhalten, falls das Lichtbogensignal (410) während der Anschnittunterstützungsphase (440) ausgegeben wurde, und anschließend für eine vorbestimmte Zeitdauer (T4) eines langen Pausenintervalls (450) die Abgabe keiner oder einer geringeren Leistung (P3) zu bewirken, bei der kein Lichtbogen auftritt,- oder, falls bis zum Erreichen einer vorgegebenen Maximaldauer (T1) der Anschnittunterstützungsphase (420) kein Lichtbogensignal (410) ausgegeben wurde, für eine vorbestimmte Zeitdauer (T2) eines Pausenintervalls (430), das kürzer ist als das besagte lange Pausenintervall, die Abgabe keiner oder einer geringeren Leistung zu bewirken, bei der kein Lichtbogen auftritt.

2. Hochfrequenzgenerator (10) nach Anspruch 1, **dadurch gekennzeichnet, dass** die Leistungssteuerung (300) so ausgebildet ist, dass die Zeitdauer des langen Pausenintervalls zwischen 500 und 1000 ms und die Zeitdauer des kurzen Pausenintervalls zwischen 100 und 400 ms beträgt.

3. Hochfrequenzgenerator (10) nach Anspruch 1 oder 2, **dadurch gekennzeichnet, dass** die Leistungssteuerung (300) so ausgebildet ist, dass die Zeitdauer einer Schnittphase 10 - 20 ms, vorzugsweise 15 ms, beträgt.

4. Hochfrequenzgenerator (10) nach einem der Ansprüche 1 bis 3, **dadurch gekennzeichnet, dass** die Leistungssteuerung (300) so ausgebildet ist, dass die Phase zur Anschnittunterstützung in jedem Falle endet und in die Schnittphase übergeht, wenn der Lichtbogendetektor (310) das Zünden eines Lichtbogens detektiert.

5. Hochfrequenzgenerator (10) nach Anspruch 4, **dadurch gekennzeichnet, dass** der Lichtbogendetektor (310) so ausgebildet ist, dass er das Zünden eines Lichtbogens durch die Detektion einer Gleichspannung feststellt.

6. Hochfrequenzgenerator (10) nach einem der Ansprüche 1 bis 5, **dadurch gekennzeichnet, dass** der Lichtbogendetektor (310) das Zünden eines Lichtbogens an einer charakteristischen Ausbildung der höheren Harmonischen erkennt.

7. Hochfrequenzgenerator (10) nach einem der Ansprüche 1 bis 6, **dadurch gekennzeichnet, dass** die Leistungssteuerung (300) so ausgebildet ist, dass die Maximaldauer der Phase zur Anschnittunterstützung weniger als 50 ms beträgt.

8. Hochfrequenzgenerator (10) nach einem der Ansprüche 1 bis 7, **dadurch gekennzeichnet, dass** die Maximaldauer der Phase zur Anschnittunterstützung und/oder die Zeitdauer der Schnittphase und/oder die Zeitdauer des langen Pausenintervalls und/oder die Zeitdauer des kurzen Pausenintervalls einstellbar ist/sind.

9. Hochfrequenzgenerator (10) nach einem der Ansprüche 1 bis 8, **dadurch gekennzeichnet, dass** die abgegebene Leistung der Phase zur Anschnittunterstützung und/oder die abgegebene Leistung der Schnittphase und/oder die abgegebene Leistung des langen Pausenintervalls und/oder die abgegebene Leistung des kurzen Pausenintervalls einstellbar ist/sind.

## Revendications

1. Générateur haute fréquence (10) pour la connexion d'un instrument électrochirurgical (210) comprenant :
- une borne de sortie électrique (125) pour un instrument électrochirurgical,
- une source (110) de courant ou de tension connectée au moins indirectement à la borne de sortie,
- un détecteur d'arc (310), qui, en fonctionnement, délivre un signal d'arc (410) représentant un arc si un arc s'est amorcé au niveau de l'instrument électrochirurgical, et
- un dispositif (300) de réglage de puissance pour régler la puissance électrique délivrée au moyen de la borne de sortie (125), le dispositif de réglage de puissance étant conçu
- premièrement pour causer la distribution d'une forte puissance de sortie (P1) pendant une phase (420) d'aide à la coupe initiale,
**caractérisé en ce que** le dispositif (300) de réglage de puissance est en outre conçu, ultérieurement à celle-ci :
- soit pour causer la distribution d'une puissance (P2) qui est réduite par rapport à la forte puissance (P1) afin de maintenir l'arc pendant une période de temps prédéterminée (T3) d'une phase de coupe (440) si le signal d'arc (410) a été émis pendant la phase (420) d'aide à la coupe initiale et, ultérieurement pendant une période de temps prédéterminée (T4) d'un long intervalle de pause (450), pour causer la distribution d'aucune puissance ou d'une plus faible puissance (P3) à laquelle aucun arc ne se produit,
- soit pour causer la distribution d'aucune puissance ou d'une faible puissance à laquelle aucun arc ne se produit, si aucun signal d'arc (410) n'a été émis jusqu'à ce qu'ait été atteinte une durée maximum prédéterminée (T1) de la phase (420) d'aide à la coupe initiale pendant une période de temps prédéterminée (T2) d'un intervalle de pause plus court que ledit long intervalle de pause.

2. Générateur haute fréquence (10) selon la revendication 1 **caractérisé en ce que** le dispositif (300) de réglage de puissance est conçu de sorte que la durée du long intervalle de pause est comprise entre 500 et 1000 ms et la durée de l'intervalle de pause court est comprise entre 100 et 400 ms.

3. Générateur haute fréquence (10) selon la revendication 1 ou 2 **caractérisé en ce que** le dispositif (300) de réglage de puissance est conçu de sorte que la durée d'une phase de coupe est de 10 à 20 ms, de préférence de 15 ms.

4. Générateur haute fréquence (10) selon l'une des revendications 1 à 3 **caractérisé en ce que** le dispositif (300) de réglage de puissance est conçu de sorte que la phase d'aide à la coupe initiale se termine dans tous les cas et passe à la phase de coupe lorsque le détecteur d'arc (310) détecte l'amorçage d'un arc.

5. Générateur haute fréquence (10) selon la revendication 4 **caractérisé en ce que** le détecteur d'arc (310) est conçu de sorte qu'il détecte l'amorçage d'un arc par la détection d'une tension cc.

6. Générateur haute fréquence (10) selon l'une des revendications 1 à 5 **caractérisé en ce que** le détecteur d'arc (310) détecte l'amorçage d'un arc à partir d'une configuration caractéristique des harmoniques supérieures.

7. Générateur haute fréquence (10) selon l'une des revendications 1 à 6 **caractérisé en ce que** le dispositif (300) de réglage de puissance est conçu de sorte que la durée maximum de la phase d'aide à la coupe initiale est inférieure à 50 ms.

8. Générateur haute fréquence (10) selon l'une des revendications 1 à 7 **caractérisé en ce que** la durée maximum de la phase d'aide à la coupe initiale et/ou la durée de la phase de coupe et/ou la durée du long intervalle de pause et/ou la durée de l'intervalle de pause court est/sont ajustable(s).

9. Générateur haute fréquence (10) selon l'une des revendications 1 à 8 **caractérisé en ce que** la puissance délivrée de la phase d'aide à la coupe initiale et/ou la puissance délivrée de la phase de coupe et/ou la puissance délivrée du long intervalle de pause et/ou la puissance délivrée de l'intervalle de pause court est/sont ajustable(s).
